# EUROPÄISCHE PATENTSCHRIFT

(11) **EP 0 482 043 B1**
(45) Veröffentlichungstag und Bekanntmachung des Hinweises auf die Patenterteilung: **28.09.1994**
(21) Anmeldenummer: 90910683.3
(22) Anmeldetag: 06.07.1990
(51) Int. Cl.: C07D 221/14, A61K 31/47

(54) **Monohydrochlorid und Monomethansulfonat von Amonafide.**
Monohydrochloride and monomethansulphonate of amonafide.
Monohydrochloride et monométhanesulfonate d'amonafide.

(30) Priorität: 11.07.1989 DE 3922771
(43) Veröffentlichungstag der Anmeldung: 29.04.1992
(73) Patentinhaber: KNOLL AG, D-67061 Ludwigshafen (DE)
(72) Erfinder: FERNANDEZ BRANA, Miguel, E-28036 Madrid (ES); CASTELLANO BERLANGA, José Maria, E-28036 Madrid (ES); SPENGLER, Reinhard, D-6700 Ludwigshafen (DE); TETZNER, Christine, D-6700 Ludwigshafen (DE)
(74) Vertreter: Karau, Wolfgang, Dr.
(86) Internationale Anmeldenummer: EP9001088
(87) Internationale Veröffentlichungsnummer: WO9100857

(56) Entgegenhaltungen:
- EP-A- 0 125 439
- DE-A- 2 423 547
- GB-A- 1 602 683
- EUR. J. MED. CHEM. - CHIMICA THERAPEUTICA, May-June, Band 16, Nr. 3, 1981

## Beschreibung

Die vorliegende Erfindung betrifft neue Salze von Amonafide.

Amonafide (Eur. J. Med. Chem. Chim. Ther. 16, 207 (1981)) ist eine Substanz, die Antitumor-Wirkung besitzt. Sie liegt in Form von sehr feinen nadelförmigen gelben Kristallen vor, die an Asbest errinnern und sich sehr schwer zu oralen Formen oder Injektionslösungen verarbeiten läßt.

Versucht man, Amonafide mit Säuren, wie Salzsäure oder Methansulfonsäure umzusetzen, so erhält man die Di-Salze, die sich wegen ihrer stark sauren Eigenschaften nicht zur Herstellung von galenischen Formen eignen. Der Grund dafür ist der, daß die Salze wegen ihres stark sauren Charakters Reizwirkungen hervorrufen. Hinzu kommt, daß sie mit vielen galenischen Hilfsstoffen, die zur Herstellung von Kapseln benötigt werden, nicht kompatibel sind.

Es wurde nun gefunden, daß bestimmte Salze von Amonafide die oben genannten negativen Eigenschaften zwar nicht besitzen, sich aber hinsichtlich der Antitumor-Wirkung nicht von Amonafide unterscheiden.

Gegenstand der Erfindung sind Salze von Amonafide der Formel 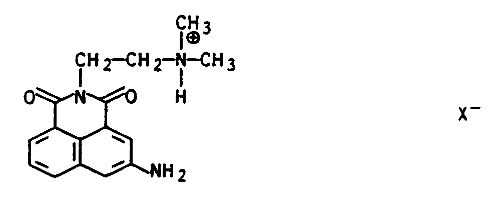
worin X⁻ ein Cl⁻- oder CH₃-SO₃- Ion ist.

Die neuen Salze lassen sich im Gegensatz zu den Di-Salzen leicht und schnell unter Bildung von im Dunkeln stabilen Lösungen lösen, und sind in fester Form leicht dosierbar, gut schüttfähig, verpreßbar und nur mäßig hygroskopisch und leicht zu trocknen. Weiter haben sie den Vorteil, daß sie mit Gelatine kompatibel sind, so daß sie leicht zu weich- und Hartgelatine-Kapseln verarbeitet werden können. Letzteres gilt insbesondere für das Monohydrochlorid.

Die Salze werden hergestellt, indem man Amonafide mit der berechneten Menge Säure umsetzt. Vorzugsweise erfolgt die Salzbildung in alkoholischer Lösung.

### Die Beispiele 1 und 2 zeigen die Herstellung der neuen Salze:

### Beispiel 1

3 g Amonafide wurden unter Rückfluß in 60 ml praktisch wasserfreiem Ethanol gelöst. Anschließend wurde tropfenweise unter starkem Schütteln 1 ml 35 %ige Salzsäure hinzugegeben. Nach dem Abkühlen wurde das entstandene Kristallisat abfiltriert und mit 10 ml wasserfreiem Ethanol gewaschen. Man erhielt 3,1 g (93 %) Amonafide-Monohydrochlorid (C₁₆H₁₈ClN₃O₂), Fp = 290°C.

### Beispiel 2

Analog Beispiel 1 erhielt man durch Umsetzen mit 0,8 ml Methansulfonsäure 3,4 g (85 %) Amonafide-Monomethansulfonat, Fp = 255°C.

Die folgenden Beispiele zeigen die guten galenische Verarbeitbarkeit der neuen Salze:

### Beispiel A

### Herstellung von Amonafide-Tabletten

| | |
|---|---|
| Amonafide·HCl | 288,4 mg |
| D-Mannit | 40,6 mg |
| Polyvinylpyrroldon | 13,0 mg |
| mikrokristalline Cellulose | 20,0 mg |
| Natrium-Carboxymethylstärke | 10,0 mg |
| hochdisperse Kieselsäure | 2,0 mg |
| Talkum | 4,0 mg |
| Magnesiumstearat | 2,0 mg |
| | 3̅8̅0̅,̅0̅ m̅g̅ |

Durch Granulieren von Wirkstoff und Mannit mit Hilfe von Polyvinylpyrrolidon wurde ein Granulat erhalten, das nach Abmischen mit den übrigen genannten Substanzen auf konventionellen Tablettenpressen gut tablettierbar war. Als Tablettenwerkzeuge dienten 11 mm R9-Rundstempel. Die Tablettenzerfallszeit betrug 9 min.

Das verwendete Polyvinylpyrrolidon besaß in einer 20 %igen wäßrigen Lösung bei 25°C die Viskosität von 20 mPa·sec.

### Beispiel B

### Herstellung von Filmtabletten

Tabletten nach Beispiel A wurden mit einem Coating folgender Zusammensetzung überzogen bis etwa 20 - 30 mg Gewichtszuwachs je Tablettenkern festzustellen war:

| | |
|---|---|
| Hydroxypropylmethylcellulose | 0,064 kg |
| Polyethylenglykol mit dem mittleren Molekulargewicht 8000 | 0,040 kg |
| Talkum | 0,080 kg |
| Titandioxid | 0,020 kg |
| Natriumlaurylsulfat | 0,002 kg |
| Ethanol | 0,794 kg |

### Beispiel C

### Herstellung von magensaftresistenten Tabletten

Tabletten nach Beispiel A wurden mit einem Coating folgender Zusammensetzung überzogen bis etwa 40 - 50 mg Gewichtszuwachs je Tablettenkern festzustellen war.

| | |
|---|---|
| Hydroxypropylmethylcellulosephthalat | 0,1620 kg |
| Dibutylphthalat | 0,0324 kg |
| Aceton | 0,8826 kg |
| Isopropanol | 0,8826 kg |
| Farbpigment | 0,0404 kg |

### Beispiel D

### Herstellung einer Injektionslösung als Konzentrat

| | |
|---|---|
| Amonafide·HCl | 564,3 mg |
| Aqua ad injectabilia ad 10 ml | |

Die Lösung wurde sterilfiltriert, in 10 ml Braunglasampullen abgefüllt und autoklaviert.

### Beispiel E

### Herstellung von Amonafide-Injektionskonzentrat als Lyophilisat

| | |
|---|---|
| Amonafide·2HCl | 2,992 kg |
| NaOH, 5 m | 1,998 kg |
| Aqua ad injectabilia ad | 20,000 kg |

Die Lösung wurde sterilfiltriert und anschließend zu je 2 ml in vials der Glasart I (Braunglas) abgefüllt und gefriergetrocknet. Der pH-Wert der rekonstituierten Lösung betrug 5,5 ± 0,5, die Osmolarität etwa 1 000 mosmol/kg, und die Restfeuchte etwa 1 %.

In dem Konzentrat lag das Amonafide als Monohydrochlorid vor.

### Beispiel F

### Herstellung von Weichgelatine-Kapseln

| | |
|---|---|
| Amonafide·HCl | 28,2 g |
| Siliconöl ad | 100,0 g |

Die homogene suspendierte Masse wurde in Weichgelatine-Kapseln der Größe 16 minims abgefüllt.

## Patentansprüche

1. Salze von Amonafide der Formel 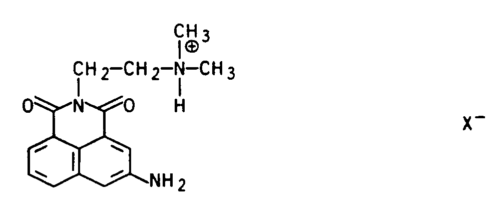 worin X⁻ ein Cl⁻- oder CH₃-SO₃⁻-Ion ist.

2. Amonafide-Monohydrochlorid.

3. Verfahren zur Herstellung der Salze gemäß Anspruch 1, dadurch gekennzeichnet, daß man Amonafide mit der berechneten Menge Salz- oder Monomethansulfonsäure umsetzt.

## Claims

1. A salt of amonafide of the formula 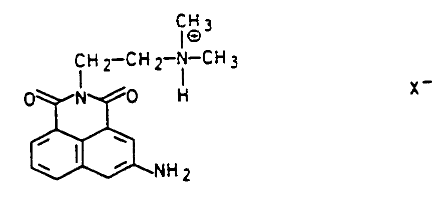 in which X⁻ is a Cl⁻ or CH₃-SO₃⁻ ion.

2. Amonafide monohydrochloride.

3. A process for the preparation of the salts as claimed in claim 1, which comprises reacting amonafide with the calculated amount of hydrochloric or mono-methanesulfonic acid.

## Revendications

1. Sels d'amonafide de la formule 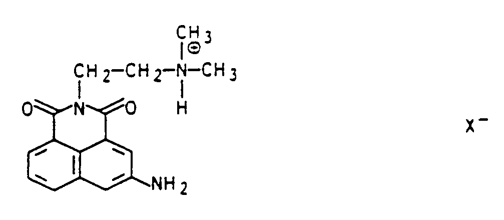 dans laquelle
X⁻ est un ion Cl⁻ ou CH₃-SO₃⁻.

2. Monochlorhydrate d'amonafide.

3. Procédé de préparation de sels selon la revendication 1, caractérisé par le fait que l'on fait réagir de l'amonafide avec la quantité calculée d'acide chlorydrique ou d'acide méthanesulfonique.
